# EUROPEAN PATENT APPLICATION

(11) **EP 2 090 281 A1**
(43) Date of publication of application: **19.08.2009**
(21) Application number: 08151187.5
(22) Date of filing: 07.02.2008
(51) Int. Cl.: A61K 8/04, A61K 8/73, A61K 8/81, A61Q 5/06

(54) **Hair styling composition**

(71) Applicant: Wella Aktiengesellschaft, 64274 Darmstadt (DE)
(72) Inventor: Birkel, Susanne, 64295 Darmstadt (DE)

(57) **Abstract**

Hair styling composition comprising a first polymeric quaternary ammonium salt, a second polymeric quaternary ammonium salt and a cosmetically acceptable carrier. This composition is suitable for achieving a hair style having long-lasting hold with high-setting properties.

## Description

### FIELD OF THE INVENTION

According to a first aspect, the present invention relates to a hair styling composition in the form of a gel comprising a first polymeric quaternary ammonium salt, a second polymeric quaternary ammonium salt, and a cosmetically acceptable carrier. According to further aspects, the present invention also relates to a hair styling kit, a method of treating hair fibers and the use of this hair styling composition. The hair styling composition, according to the present invention, may be used for achieving a hair style having long-lasting hold with high-setting properties.

### BACKGROUND OF THE INVENTION

Hair styling compositions are intended to be used for achieving individual hair styles, and for temporarily holding hairs in place for a period of time.

These compositions may be provided in various forms such as mousse, gel and wax. Hair styling compositions in the form of a gel, particularly compositions in the form of a transparent gel, are popular with consumers and/or users. These compositions are usually easy to apply onto hairs, e.g. as a hand gel or as a spray gel, and they have appealing aesthetics, particularly when being transparent.

Hair styling compositions usually comprise at least one hair-fixing polymer. Many hair-fixing polymers are available, including anionic hair-fixing polymers, amphoteric hair-fixing polymers, non-ionic hair-fixing polymers and cationic hair-fixing polymers. It is already available various compositions comprising specific combinations of hair-fixing polymers, these compositions providing various degrees of setting, e.g. from natural to ultra strong hold, and various hold factors, e.g. from short term to long term (more than 24 h) to the hair style.

There is a constant need, however, for providing a hair styling composition, preferably a composition in the form of a gel, intended to be used for achieving individual hair styles having long-lasting hold with high-setting properties. An improved degree of setting and/or an improved hold factor may be achieved by increasing the proportion of hair-fixing polymers into the composition. Nevertheless, the increase of the proportion of these polymers into a composition in the form of a gel may impair the application experience. When used as a hand gel, the composition may leave sticky residues onto skin and/or hairs. When dispensed as a spray gel, e.g. by using a spray dispenser, the composition may not be evenly sprayed and may leave residues onto hairs. In addition, such incorporation may impair the aesthetics, such as the transparency of the composition, this composition turning milky and/or yellowish. There is a need, thereof, for providing a hair styling composition in the form of a gel intended to be used for achieving a hair style having long-lasting hold with high-setting properties while being easy to apply onto hairs. There is also a need for providing a composition in the form of a gel intended to be used for achieving a hair style having long-lasting hold with high-setting properties without impairing its aesthetics.

There is also a constant need for providing a hair styling composition, particularly a composition in the form of a gel, being safer for both the user and the environment. Particularly, it is recommended to limit the proportion of alcohol, e.g. ethanol and/or isopropanol, being incorporated into such composition. Nevertheless, lowering the proportion of these alcohols may impair the degree of setting and/or the hold factor conferred by a composition. There is a need, therefore, for providing a composition in the form of a gel being substantially free of alcohol while being used for achieving a hair style having long-lasting hold with high-setting properties.

### SUMMARY OF THE INVENTION

According to a first aspect, the present invention relates to a hair styling composition in the form of a gel, comprising:
(a) a first polymeric quaternary ammonium salt obtainable by copolymerization of monomers of N-vinylimidazole, N-vinylpyrrolidone, methacrylamide and quaternized N-vinylimidazole;
(b) a second polymeric quaternary ammonium salt of hydroxyethylcellulose quaternized with diallyldimethyl ammonium chloride; and,
(c) a cosmetically acceptable carrier.

According to a second aspect, the present invention relates to a hair styling kit comprising:
(a) a hair styling composition, according to the first aspect of the invention; and,
(b) a dispenser, which dispenser comprises:
   i) a container comprising said hair styling composition; and,
   ii) a dispensing pump, which is mounted onto said container;
wherein, in use, said dispenser is adapted to dispense, upon actuation of said dispensing pump, the hair styling composition as a spray.

According to a third aspect, the present invention relates to a method of treating hairs comprising the steps of:
(1) providing a hair styling composition, according to the first aspect of the invention;
(2) applying said composition onto hairs; and,
(3) achieving the hair style.

According to a fourth aspect, the present invention relates to the use of a hair styling composition, according to the first aspect of the invention, for achieving a hair style having long-lasting hold with high-setting properties.

As used herein, the term "gel" means a composition having a viscosity from 30 mm²/s to 50,000 mm²/s, measured at 25°C using the device Kinematic Viscosity® (capillary 52020) from Cannon Fenske.

As used herein, the term "spray" means a composition being dispensed, e.g. by using a spray dispenser, in the form of a multitude of finely divided droplets.

As used herein, the term "substantially free" means that the composition comprises less than 0.01%, preferably 0%, of a component.

As used herein, the term "3 point bending force" means the degree of setting conferred to the hair style by applying a hair styling composition onto hairs.

As used herein, the term "long-lasting hold" means the hold factor conferred by the hair style over a period of time and/or under determined conditions of humidity, by applying a hair styling composition onto hairs.

As used herein, the terms "weight percentage" and "percentage by weight" of a component mean the percentage of active component and not the percentage of the raw material comprising this active, unless otherwise specified (see examples).

All percentages, ratios and proportions herein are by weight, unless otherwise specified.

### DETAILED DESCRIPTION OF THE INVENTION

According to the first aspect, the present invention relates to a hair styling composition in the form of a gel, comprising a first polymeric quaternary ammonium salt; a second polymeric quaternary ammonium salt; and, a cosmetically acceptable carrier.

The hair styling composition, according to the present invention, is in a form of a gel. This composition is preferably a transparent gel, more preferably a transparent gel having a turbidity value from 0 NTU to 2000 NTU, still more preferably a transparent gel having a turbidity value from 0 NTU to 200 NTU. The transparency of the composition may be measured using the Laboratory Turbidity Photometer LTP5 (with a 50 mm cuvette (glass); position normal; uncolored) from D. Lange GmbH.

The composition, according to the present invention, comprises a first polymeric quaternary ammonium salt obtainable by copolymerization of monomers of N-vinylimidazole, N-vinylpyrrolidone, methacrylamide and quaternized N-vinylimidazole. This first salt comprises, therefore, monomers of N-vinylimidazole, N-vinylpyrrolidone, methacrylamide and quaternized N-vinylimidazole. This first quaternary ammonium salt is a hair-fixing polymer.

This composition comprises preferably a first polymeric quaternary ammonium salt obtainable by copolymerization of monomers of N-vinylimidazole, N-vinylpyrrolidone, methacrylamide and 3-methyl-1-vinylimidazolium methylsulfate. This preferred salt comprises, therefore, monomers of N-vinylimidazole, N-vinylpyrrolidone, methacrylamide and 3-methyl-1-vinylimidazolium methylsulfate. The INCI name of this preferred first salt is polyquaternium-68. A commercially available example of this preferred first salt includes Luviquat Supreme® from BASF Corporation.

The composition may comprise from 0.1% to 15%, preferably from 2.5% to 11%, more preferably from 4% to 10%, of the first polymeric quaternary ammonium salt, by weight of the total composition.

The composition, according to the present invention, comprises a second polymeric quaternary ammonium salt of hydroxyethyl cellulose quaternized with diallyldimethyl ammonium chloride. The INCI name of this second salt is polyquaternium-4. A commercially available example of this second salt includes Celquat LV® from National Starch & Chemical Company. This second quaternary ammonium salt is a hair-fixing polymer.

The composition may comprise from 0.1% to 10%, preferably from 0.15% to 5%, more preferably from 0.5% to 3%, of the second polymeric quaternary ammonium salt, by weight of the total composition.

The composition may comprise a weight ratio of first polymeric quaternary ammonium salt : second polymeric quaternary ammonium salt from 1 : 5 to 15 : 1, preferably from 1 : 2 to 5 : 1.

The inventors have surprisingly found that the incorporation of the first and the second polymeric quaternary ammonium salts into the composition allows, once applied onto hairs, achieving a hair style having long-lasting hold with high-setting properties, without impairing neither the application experience nor the aesthetics of the composition. Particularly, the inventors have found that the composition in the form of the gel exhibits no milky and/or yellowish appearance. In addition, the composition leaves no sticky residues onto skin and/or hairs. Finally, the composition is easily and evenly applying onto hairs, particularly when dispensed as a spray by using a spray dispenser. Without wishing to be bound by theory, it is believed that the first and the second polymeric quaternary ammonium salts are highly compatible to each other, particularly due to their charge and their chemical structure. It is also believed that such compatibility prevents these quaternary ammonium salts to precipitate and/or to agglomerate, and prevents, therefore, the alteration of the transparency of the composition. It is also believed that the easy and even application of the composition is facilitated by the charge of these quaternary ammonium salts, such charge matching the charge of hairs.

The composition, according to the present invention, comprises a cosmetically acceptable carrier. This carrier may be selected from either an aqueous medium or an aqueous-alcoholic medium. Preferably, the carrier is an aqueous medium.
When the carrier is an aqueous-alcoholic carrier, this carrier comprises water and an alcohol. Alcohols are usually incorporated into the hair styling compositions to improve the stability of the composition over time and to improve the hold factor confers to the hair-style by applying this composition onto hairs. The alcohol is preferably selected from alcohols having from one to five carbon atoms, more preferably from ethanol, isopropanol, n-propanol, ethylene glycol, glycerol, propylene glycols, or mixtures thereof, still more preferably from ethanol, isopropanol or mixtures thereof. This aqueous-alcoholic carrier comprises preferably from 0.01 % to 10%, more preferably from 0.01% to 5%, still more preferably from 0.01% to 2%, alcohol, by weight of carrier. This carrier also comprises preferably from 90% to 99.9%, more preferably from 95% to 99.9%, still more preferably from 98% to 99.9%, water, by weight of carrier.

When the carrier is an aqueous carrier, this carrier consists essentially of water and is substantially free of alcohol. Preferably, the carrier is water. The inventors have surprisingly found that high-setting properties and a long-lasting hold are achieved with the composition, according to the present invention, even when the composition is substantially free of alcohol. The composition comprising both quaternary ammonium salts and being substantially free of alcohol performs equally, or even better, than a composition comprising a significant proportion of alcohol. Without wishing to be bound by theory, it is believed that the properties of both quaternary ammonium salts for conferring long-lasting hold and high-setting properties to a hair-style are independent to the presence or the absence of alcohols into the composition.

The composition comprises from 80% to 99%, preferably from 85% to 95%, more preferably from 87% to 92%, of a cosmetically acceptable carrier, by weight of the total composition.

The composition has preferably a viscosity from 30 mm²/s to 50,000 mm²/s, more preferably from 30 mm²/s to 1000 mm²/s, more preferably from 30 mm²/s to 100 mm²/s, at 25°C. This viscosity is measured using the device Kinematic Viscosity® (capillary 52020) from Cannon Fenske. The provision of a composition having a viscosity from 30 mm²/s to 1000 mm²/s is advantageous as this composition is particularly suitable to be dispensed as a spray, for example by using a spray dispenser comprising a dispensing pump adapted to dispense, upon actuation, this composition as a spray. When dispensed as a spray, this composition is dispensed in a multitude of finely divided droplets, these droplets being easily and evenly distributed over the hairs without leaving any residue. A used herein, the term "finely divided droplets" means droplets of hair styling composition being substantially spherical in shape and having a diameter of about 130 to 500 µm.

The composition has preferably a pH from 5 to 7.5, more preferably 6 to 7, still more preferably from 6.1 to 6.5. The provision of a composition having a pH 6.1 to 6.5 is particularly advantageous as this pH matches the pH of skin and hairs without impairing the properties of the first and second quaternary ammonium salts as hair-fixing polymers. A desirable pH can be achieved and/or stabilized by using conventional buffers and pH-adjusting agents.

The composition may further comprise a surfactant. The composition preferably comprises from 0.3 % to 2 %, more preferably from 0.5% to 1.9%, still more preferably from 0.9 % to 1.5 %, of a surfactant, by weight of the total composition. When present, the surfactant is useful for emulsifying hydrophobic components, e.g. hydrophobic perfume oils, in order not to impair the transparency of the composition.

Suitable surfactants include any surfactant known to those skilled in the art as suitable for incorporation into a hair care composition. Suitable examples of surfactants may be found in the CTFA International Cosmetics Ingredient Dictionary and Handbook, "Surfactants", 10th edition (2004). The surfactant may be selected from non-ionic surfactants, anionic surfactants, cationic surfactants, amphoteric surfactants, zwitterionic surfactants or mixtures thereof. The surfactant is selected preferably from non-ionic surfactants, more preferably from ethoxylated fatty alcohols, ethoxylated fatty acids, ethoxylated fatty acid glycerides, ethoxylated alkylphenols, ethoxylated castor oil, ethoxylated hydrogenated castor oil, fatty acid sugar esters, fatty acid sorbitan esters, polyglyceryl fatty acid esters, alkylglucosides, alkyloligoglucosides, alkylpolyglucosides, or mixtures thereof; still more preferably from ethoxylated castor oil, ethoxylated hydrogenated castor oil, or mixtures thereof.

Castor oil and hydrogenated castor oil are preferably castor oils with a degree of ethoxylation from 10 to 100, more preferably from 20 to 60. The INCI name of these castor oils is usually PEG-x castor oil or PEG-x hydrogenated castor oil, wherein x is the degree of ethoxylation. Examples of commercially available ethoxylated hydrogenated castor oils include Cremophor CO 40® (PEG-40 hydrogenated castor oil) and Cremophor CO 60® (PEG-60 hydrogenated castor oil) from BASF Corporation.

The composition may further comprise a thickening polymer. The composition preferably comprises from 0.01 % to 5%, more preferably from 0.1% to 3%, still more preferably from 0.2% to 2%, of a thickening polymer, by weight of the total composition.

Suitable thickening polymers include any thickening polymer known to those skilled in the art as suitable for incorporation into a hair care composition. Suitable examples of thickening polymers may be found in the CTFA International Cosmetics Ingredient Dictionary and Handbook, "Viscosity-increasing agents - Aqueous"; "Viscosity-increasing agents - Nonaqueous"; "Suspending agents - Nonsurfactants", 10th edition (2004).

The thickening polymer may be selected from natural thickening polymers, synthetic thickening polymers or mixtures thereof. This thickening polymer is preferably soluble in the cosmetically acceptable carrier.

The synthetic thickening polymer is preferably selected from polymers comprising monomers of acrylic acid, its salts or derivatives. A non-limiting list of suitable synthetic thickening polymers includes acrylates/C10-30 alkyl acrylate crosspolymers (e.g. crosspolymer commercially available as Pemulen TR-1 Polymer® from Noveon, Inc.), acrylates/steareth-20 itaconare copolymers (e.g. Structure 2001® from National Starch & Chemical Company), acrylates/Ceteth-20 itaconate copolymers (e.g. Structure 3001® from National Starch & Chemical Company), acrylates/aminoacrylates/C10-30 alkyl PEG-20 itaconate copolymers (e.g. Supreme Plus from National Starch & Chemical Company), Carbomer (e.g. Carbopol 980 Polymer® from Noveon, Inc.), acrylate copolymers (e.g. Carbopol Aqua SF-1® from Noveon, Inc.), ammonium acryloyldimethyltaurate/VP copolymers (e.g. Aristoflex AVC® from Clariant Corporation), Ammonium acryloyldimethy;taurate/Beheneth-25 methacrylate crosspolymers (e.g. Aristoflex HMB® from Clariant Corporation), polyacrylate-1 crosspolymers (e.g. Carbopol Aqua CC Polymer® from Noveon, Inc.), acrylates/steareth-20 methacrylate crosspolymers (e.g. Aculyn 88 Polymer® from Rohm and Hass Company, Inc.), acrylates/beheneth-25 methacrylate copolymers (e.g. Aculyn 28 Polymer® from Rohm and Hass Company, Inc.), acrylates/steareth-20 methacrylate copolymers (e.g. Aculyn 22® from Rohm and Hass Company, Inc.), acrylic acid/VP crosspolymers (e.g. Ultrathix P-100® from International Specialty Products), and hydroxypropyl guar (e.g. Jaguar HP-105® from Rhodia Inc.).

A non-limiting list of natural thickening polymers includes guar gums, xanthan gums (e.g. Keltrol® from CP Kelco), corn starch modified (e.g. Amaze® from National Starch & Chemical Company), carrageenan gums (e.g. Viscarin GP209, NF® from FMC Corporation), and hydroxyethylcellulose (e.g. Natrosol Hydroethylcellulose® from Hercules Incoprorated).

The composition may further comprise particles. When present, the composition preferably comprises from 0.005% to 5%, more preferably from 0.01% to 3%, particles, by weight of the total composition.

The particles may be selected from particles comprising at least one polyolefin, silica particles, or mixtures thereof. The particles comprising at least one polyolefin preferably comprise polyethylene, polypropylene or mixtures thereof. An example of commercially available particles comprising at least one polyolefin includes Microthene FN-510-00 from Equistar Chemicals, LP. The silica particles preferably comprise colloidal silica, filmed silica, precipitated silica or silica gels. An example of commercially available silica particles includes Sipernat 22LS from Degussa AG.

The composition may further comprise an additional hair-fixing polymer. As used herein, "an additional hair-fixing polymer" means a hair-fixing polymer other than the first polymeric quaternary ammonium salt and the second polymeric quaternary ammonium salt. When present, the composition comprises preferably from 0.1% to 5%, more preferably from 0.1% to 2%, of an additional hair-fixing polymer, by weight of the total composition.

Preferably, the additional hair-fixing polymer is selected from anionic hair-fixing polymer, amphoteric hair-fixing polymer, cationic hair-fixing polymer, or mixtures thereof. Suitable additional hair-fixing polymers may be found in the CTFA International Cosmetics Ingredient Dictionary and Handbook, "Hair fixatives", 10th edition (2004). The additional cationic hair-fixing polymer may be selected from polyquaternium-6; polyquaternium-7; polyquaternium-10; polyquaternium-11; polyquaternium-16; polyquaternium-28; polyquaternium-35; polyquaternium-37; polyquaternium-47; polyquaternium-57; polyquaternium-80; copolymers of polyvinylpyrrolidone and imidazolimine methochloride; terpolymer of dimethyldiallyl ammonium chloride, sodium acrylate and acrylamide; terpolymer of vinylpyrrolidone, dimethyl aminoethyl methacrylate and vinyl caprolactam; quaternary ammonium salt of hydroxyethyl cellulose and a trimethylammonium substituted epoxide; or mixtures thereof. The amphoteric hair-fixing polymer may be selected from copolymers of octoylacrylamide, t-butylaminoethylmethacrylate and two or more monomers, comprising acrylic acid, methacrylic acid or their esters; isobutylene/ethylmaleimide/hydroxyethylmaleimide copolymer; or mixtures thereof.

The composition may be substantially free of a non-ionic hair-fixing polymer.

The composition may also be substantially free of propellant. Preferably, the composition may be substantially free of propellant selected from dimethyl ether, propane, butane or mixtures thereof.

A variety of additional optional ingredients may be incorporated into the composition of the present invention. Non-limiting examples of these additional ingredients include fragrances and perfume oils; preservatives, such as parabene, iodopropynyl butylcarbamate, or mixtures thereof; moisturiser; opacifying agents, such as ethylene glycol distearate, fatty acid monoalkylolamide, or mixtures thereof; buffer substances, such as sodium citrate, sodium phosphate, or mixtures thereof; hair care substances, such as betaine, panthenol, plant extracts, vegetable extracts, protein hydrolysates, silk hydrolysates, lanolin derivatives, or mixtures thereof; physiologically compatible silicone derivatives, such as volatile or non-volatile silicone oils, high molecular weight siloxane polymers or mixtures thereof; light protective agents; antioxidants; radical-trapping agents; antidandruff agents; fatty alcohols; vitamins; direct dye compounds; luster-imparting substances; and, pigments.

According to a second aspect, the present invention relates to a hair styling kit comprising a hair styling composition, according to the first aspect, and a dispenser. The dispenser comprises a container, which container comprising the hair styling composition, and a dispensing pump. The dispensing pump may be mounted directly or indirectly, e.g. via a sealing means, onto the container and it is in fluid communication with the container. This dispenser is adapted to dispense, upon actuation of said dispensing pump, the hair styling composition as a spray. Such dispensers, used for dispensing hair gel compositions in the form of a gel as a spray, are known from the skilled person.

The provision of a kit, which is suitable for dispensing the hair styling composition as a spray, is advantageous as it allows an easy and even application of this composition onto the hairs. It also allows working on damp hairs to achieve superior hold. The composition, according to the first aspect, is particularly suitable to be stored into, and dispensed via, such dispenser and this composition is easily dispensed in a multitude of finely divided droplets, allowing an even distribution of the composition over the hairs.

According to a third aspect, the present invention relates to a method for treating hairs comprising the steps of:
(1) providing a hair styling composition, according to the first aspect of the invention;
(2) applying this composition onto hairs; and,
(3) achieving the hair style.

The composition may be applied as a hand gel or as a spray gel. When applied as a hand gel, the method may comprise the steps of dispensing this composition into the palm of one hand, rubbing both hands altogether to spread this composition onto palms, and then rubbing and/or massaging the hairs with the palms to apply this composition evenly onto hairs. When applied as a spray gel, the method may comprise the steps of actuating the dispenser pump for dispensing the composition as a spray and, simultaneously, directing the spray to allow an even application of the composition onto hairs.

It is preferably applied from 15 to 45 mg (per gram of hair), more preferably from 20 to 40 mg (per gram of hair), still more preferably from 25 to 35 mg (per gram of hair), of the composition onto hairs. This is advantageous for achieving a hair style having long-lasting hold with high-setting properties.

The composition is preferably applied onto hair as a spray. The composition may be applied using a hair styling kit, according to the second aspect of the invention. The spraying of the composition onto hairs is advantageous as it allows an easy and even distribution onto hairs of the composition.

The hair style may be achieved by working hairs with fingers till the desired hair style is obtained.

The method may further comprise the step of applying an aqueous medium onto hairs, before applying the hair styling composition onto hairs. The aqueous medium is preferably water, more preferably tap water. This medium may be applied onto hairs by wetting hands and then massaging evenly hairs with the hands. The medium may also be applied onto hairs by spraying this medium onto hairs, e.g. by using a spray dispenser,. The medium may also be applied by wetting the hairs with tap water, then shampooing the hairs with a conventional shampoo, then rinsing-off the hairs with water and removing the water in excess by massaging the hairs with a towel. The application of an aqueous medium before applying the composition is advantageous as the application of the hair styling composition is facilitated, allowing an even distribution, all over the hairs of the composition. The application of an aqueous medium before applying the composition is also advantageous as it increases the adhesion the first and second quaternary ammonium salts onto hairs, and so it increases the degree of setting of the hair style.

The method may further comprise the step of blow-drying hairs. Hairs may be blow-dried by using a conventional blow-drier. This step is preferably performed simultaneously to the step of achieving the hair style.

This method is particularly suitable for achieving a hair-style having long-lasting hold with high-setting properties.

A hair style with high-setting properties has preferably a 3 point bending force from 0.1 N to 10 N, preferably from 1.8 N to 6 N.

The hair style having long-lasting hold has preferably a hold factor from 30% to 100%, preferably from 70%, to 100% after 24 h. The provision of a composition having a hold factor from 30% to 100% after 24 h is advantageous in order to achieve a sufficient hold over a period of time, preferably for at least 5 h, and more preferably for at least 24 h.

According to a fourth aspect, the present invention relates to the use of the hair styling composition, according to the first aspect of the invention, for achieving a hair style having long-lasting hold with high-setting properties.

### Methodology

The setting and the hold conferred to a hair style by applying a hair styling composition onto hairs can be determined by measuring, respectively, the 3 point bending force and the hold factor.

The 3 point bending force is measured according to the methodology detailed in F. Frosch, F. Vogel, 6th International Hair Science Symposium Of the German Wool Research Institute, Luneburg/Germany (1988). See also the methodology DIN-EN-658-5 from the American National Standards Institute.
The hold factor is measured according to the methodology developed by the Institute Fresenius, Germany and detailed in C.R. Robbins, Chemical and Physical Behavior of Human Hair, 3rd edition, page 352, Springer-Verlag, New York (1994).

### Examples

The following examples further describe and demonstrate the preferred embodiments within the scope of the present invention. The examples are given solely for the purpose of illustration, and are not to be construed as limitations of the present invention since many variations thereof are possible without departing from its scope.

All weights provided in these examples are weights of the commercially available materials, including active(s) and/or solvent and/or by-products.

### The compositions in the form of a gel (see table 1) are prepared as follows:

**Table 1**

| Examples | 1 | 2 | 3 | 4 | 5 | 6 |
|---|---|---|---|---|---|---|
| Ingredients (%w/w) | | | | | | |
| Water ¹ | Qsp | Qsp | Qsp | Qsp | Qsp | Qsp |
| Polyquaternium-68 ² | 6.75 | 6.75 | 6.0 | 7.0 | 6.75 | 6.0 |
| Polyquaternium-4 ³ | 1.35 | 1.35 | 1.6 | 1.25 | 1.3 | 1.0 |
| PEG-40 hydrogenated castor oil ⁴ | 0.8 | 0.8 | --- | 0.6 | 0.4 | 0.4 |
| PEG-60 hydrogenated castor oil ⁵ | 0.4 | 0.4 | --- | 0.6 | 0.8 | --- |
| Polyacrylate-1 crosspolymer ⁶ | --- | --- | --- | --- | 0.25 | 3.0 |
| Glycolic acid ⁷ | --- | --- | 0.05 | --- | --- | --- |
| Acrylates/beheneth-25 methacrylate copolymer ⁸ | --- | --- | --- | --- | --- | 0.6 |
| Hydroxyethylcellulose ⁹ | --- | --- | --- | 0.2 | --- | 0.4 |
| Phenoxyethanol & ethylhexylglycerin ¹⁰ | 0.4 | 0.4 | 0.5 | --- | --- | --- |
| PHB-methylester ¹¹ | 0.2 | 0.2 | 0.2 | 0.2 | 0.2 | 0.4 |
| DMDMH ¹² | 0.02 | 0.02 | 0.02 | 0.05 | 0.1 | 0.1 |
| Disodium EDTA ¹³ | 0.13 | 0.13 | 0.05 | 0.05 | 0.08 | --- |
| 2-amino-2-methyl-1-propanol ¹⁴ | --- | --- | --- | --- | --- | 0.2 |
| Polyethylene particles ¹⁵ | --- | 0.09 | --- | --- | --- | --- |
| Silica particles ¹⁶ | --- | 0.49 | --- | --- | --- | --- |
| Fragrance | 0.15 | 0.15 | --- | 0.18 | 0.20 | 0.25 |

| | | | | | | |
|---|---|---|---|---|---|---|
| 1: Deionized water ; 2: Luviquat Supreme® from BASF Corporation; 3: Celquat LV® from National Starch & Chemical Company; 4: Cremophor C040® from BASF Corporation; 5: Cremophor CO60® from BASF Corporation; 6: Carbopol Ultrez 21 Polymer from Noveon Inc.; 7: Glycolic acid from Merck-Schuchardt OHG; 8: Aculyn 28 Polymer from Rohm and Hass Company, Inc.; 9: Natrosol Hydroxyethylcellulose® from Hercules Incorporated; 10: Euxyl PE 9010 from Schulke & Mary GmBH; 11: PHB-methylester from Schutz & Co.; 12: Nippaguard DMDMH from Clariant; 13: Disodium EDTA from BASF; 14: AMP Ultra PC from Angus Chemical Company; 15: Sipernat 22LS from Degussa AG; 16: Microthene FN-510-00 from Equistar Chemicals, LP. | | | | | | |

Examples 1 to 5 are particularly suitable for being dispensed as spray gels, e.g. by using a spray dispenser. Example 5 may also be used as a hand gel. Example 6 is particularly suitable to be used as a hand gel.

Table 2 details the 3 bending point force and the hold factor after 5 h and 24 h of the hair style, after applying the compositions of examples 1 and 4.

**Table 2**

| Parameters \ Examples | Example 1 | Example 4 |
|---|---|---|
| 3 bending point force [N] | 2.03 +/- 0.4 | 5.0 +/- 0.4 |
| Hold factor after 5 hours [%] | 44.5 +/- 5.8 | 83.12 +/-3.5 |
| Hold factor after 24 hours [%] | 33.0 +/- 2.2 | 74.9 +/- 6.1 |

| | | |
|---|---|---|
| Both compositions, according to examples 1 and 4, exhibit an ultra strong and long-term hold. | | |

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

## Claims

1. A hair styling composition in the form of a gel, comprising:
(a) a first polymeric quaternary ammonium salt obtainable by copolymerization of monomers of N-vinylimidazole, N-vinylpyrrolidone, methacrylamide and quaternized N-vinylimidazole;
(b) a second polymeric quaternary ammonium salt of hydroxyethylcellulose quaternized with diallyldimethyl ammonium chloride; and,
(c) a cosmetically acceptable carrier.

2. A composition, according to claim 1, comprising:
(a) from 0.1% to 15% of the first polymeric quaternary ammonium salt, by weight of the total composition; and,
(b) from 0.1% to 10% of the second polymeric quaternary ammonium salt, by weight of the total composition.

3. A composition, according to any of the preceding claims, comprising from 80% to 99% of a cosmetically acceptable carrier, by weight of the total composition.

4. A composition, according to any of the preceding claims, having a viscosity from 30 mm²/s to 50,000 mm²/s at 25°C.

5. A composition, according to any of preceding claims, comprising from 0.3% to 2% of a surfactant, by weight of the total composition.

6. A composition, according to any of preceding claims, comprising from 0.01% to 5% of a thickening polymer, by weight of the total composition.

7. A composition, according to any of the preceding claims, comprising from 0.005% to 5% of a particulate material, by weight of the total composition.

8. A hair styling kit comprising:
(a) a hair styling composition, as defined in claims 1 to 7; and,
(b) a dispenser, which dispenser comprises:
i) a container comprising said hair styling composition; and,
ii) a dispensing pump, which is mounted onto said container;
wherein, in use, said dispenser is adapted to dispense, upon actuation of said dispensing pump, the hair styling composition as a spray.

9. Method of treating hairs comprising the steps of:
(a) providing a hair styling composition, as defined in claims 1 to 7,
(b) applying said composition onto hairs; and,
(c) achieving the hair style.

10. Use of the hair styling composition, as defined in claims 1 to 7, for achieving a hair style having long-lasting hold with high-setting properties.
